# EUROPÄISCHE PATENTSCHRIFT

(11) **EP 0 354 441 B1**
(45) Veröffentlichungstag und Bekanntmachung des Hinweises auf die Patenterteilung: **17.05.1995**
(21) Anmeldenummer: 89114143.4
(22) Anmeldetag: 01.08.1989
(51) Int. Cl.: C12Q 1/26, C12Q 1/32, C12Q 1/54

(54) **Verfahren zur kolorimetrischen Bestimmung eines Analyten mittels enzymatischer Oxidation**
Method for the colorimetric determination of analyte using enzymatic oxydation
Procédé de détermination colorimétrique d'analyse utilisant l'oxidation enzymatique

(30) Priorität: 09.08.1988 DE 3826922
(43) Veröffentlichungstag der Anmeldung: 14.02.1990
(73) Patentinhaber: BOEHRINGER MANNHEIM GMBH, 68298 Mannheim (DE)
(72) Erfinder: Hoenes, Joachim, Dr., D-6940 Weinheim (DE)

(56) Entgegenhaltungen:
- EP-A- 0 120 440
- EP-A- 0 296 481
- DE-A- 3 820 404
- US-A- 4 105 800
- DATABASE WPIL/DERWENT, 1987, Derwent Publications Ltd., London (GB); no. 87062511 (09)#

## Beschreibung

Die Erfindung betrifft ein Verfahren zur kolorimetrischen Bestimmung eines Analyten mittels enzymatischer Oxidation des Analyten in Gegenwart eines Elektronenakzeptors und Bestimmung des reduzierten Elektronenakzeptors durch Farbbildung als Maß fur die Menge des Analyten.

Weiter betrifft die Erfindung ein Mittel zur kolorimetrischen Bestimmung eines Analyten durch enzymatische Oxidation des Analycen, enthaltend eine Oxidoreduktase und einen farbbildenden Elektronenakzeptor.

Die Erfindung erstreckt sich speziell auf die Verwendung einer aromatischen Nitrosoverbindung als direkten farbbildenden Elektronenakzeptor eines Analyt / Oxidase-Systems oder eines Analyt / PQQ-abhängigen Dehydrogenase-Systems.

Enzymatische Oxidationen ermoglichen in der Analytik den Nachweis und die Bestimmung von Substanzen in verschiedensten Probenmaterialien. Hierbei wirkt ein oxidierendes Enzym in Gegenwart eines die Elektronen der Oxidationsreaktion aufnehmenden Akzeptors auf ein entsprechendes Enzymsubstrat ein. Die Reduktion des Elektronenakzeptors zeigt die Anwesenheit des Enzymsubstrats an. Hierbei hat es sich bisher als besonders vorteilhaft erwiesen, wenn der reduzierte Elektronenakzeptor durch Farbbildung nachgewiesen werden kann, da dies nicht notwendigerweise nur mittels teurer Meßapparaturen möglich ist, sondern gegebenenfalls auch visuell erfolgen kann.

Bekannte Methoden zur kolorimetrischen Bestimmung von Substanzen mittels oxidierend wirkender Enzyme verwenden Oxidasen oder Dehydrogenasen. Beide Enzymgruppen gehören zur Hauptgruppe der Oxidoreduktasen (Römpps Chemie-Lexikon, Franckh-sche Verlagshandlung, Stuttgart, 8. Auflage, 1985, Band 4, Seite 2952; Lexikon Biochemie, Hrsg. H. D. Jakubke, Verlag Chemie, Weinheim, 2. Auflage, 1981, Seite 194), deren Mitglieder nach ihren natürlichen Elektronenakzeptoren unterschieden werden können.

Natürlicher Elektronenakzeptor für Oxidasen ist molekularer Sauerstoff (Römpps Chemie-Lexikon, Franckh-sche Verlagshandlung, Stuttgart, 8. Auflage, 1985, Band 4, Seite 2946). Stellvertretend für den Stand der Technik des Einsatzes von Oxidasen zur kolorimetrischen Bestimmung von Analyten sei A. Kunst et al. in "Methods in enzymatic analysis", Hrsg. H. U. Bergmeyer, Verlag Chemie, Weinheim, 3. Auflage, 1984, Band 6, Seite 178 - 185 zitiert. Glucose wird dort in Serum, Plasma oder deproteinisiertem Blut durch Umsetzung mit Glucose-Oxidase und Luftsauerstoff in wässriger Lösung nachgewiesen, indem das bei dieser Reaktion durch Reduktion des Sauerstoffs gebildete Wasserstoffperoxid in Gegenwart von Peroxidase oxidierend und damit farbbildend auf ebenfalls in der Reaktionsmischung vorliegendes Phenol und 4-Aminophenazon wirkt. Als Fehlerquelle wird in dieser Literaturstelle selbst die Anwesenheit von reduzierend wirkenden Substanzen, wie Ascorbinsäure, Harnsäure oder Glutathion genannt. Störend wirken außerdem Übergangsmetallionen oder Häm und Häm-Proteine, wie sie leicht in von Blut abgeleiteten Proben auftreten können, weil sie Wasserstoffperoxid zersetzen. Auch Probeninhaltsstoffe, die mit Wasserstoffperoxid und Peroxidase und gegebenenfalls weiteren im Nachweisreagenz enthaltenen Substanzen, wie z. B. Phenol oder 4-Aminophenazon zu einer Farbbildung führen, können zu falschen Resultaten führen. Solche Stoffe können z. B. Bilirubin oder auch Medikamente, wie z. B. -Methyldopa sein, die durchaus in von Blut abgeleiteten Proben oder in Urin vorkommen können.

In J. Siedel et al. in "Methods in enzymatic analysis", Hrsg. H. U. Bergmeyer, Verlag Chemie, Weinheim, 3. Auflage, 1984, Band 8, Seite 139 - 148, wird die kolorimetrische Bestimmung von Gesamtcholesterin in Serum oder Plasma derart beschrieben, daß zunächst estergebundenes Cholesterin mit Cholesterin-Esterase freigesetzt wird. Dann wird Cholesterin durch Umsetzung mit Cholesterin-Oxidase und Luftsauerstoff in wäßriger Lösung bestimmt, wobei das bei dieser Reaktion gebildete Wasserstoffperoxid in Gegenwart von Peroxidase oxidierend und damit farbbildend auf ebenfalls in der Reaktionsmischung vorliegendes Phenol und 4-Aminoantipyrin wirkt. Die Farbbildung ist ein Maß für die Menge an Gesamtcholesterin in der Probe.

Sämtliche für das vorstehend genannte Verfahren zur Bestimmung von Glucose mittels Oxidase genannten Nachteile treffen in gleichem Maße für die beschriebene Cholesterin-Bestimmungsmethode zu. Diese Nachteile sind unabhängig davon, ob die Nachweisreaktion z. B. in einer Küvette oder auf einem Trockenreagenzträger, wie sie z. B. aus EP-A-0 016 387, DE-A-32 47 608, EP-A-0 262 445 oder EP-A-0 256 806 bekannt sind, durchgeführt wird. Insbesondere bei Durchführung der vorgenannten Bestimmungsmethoden auf festen Trägern, in sogenannten Trockentests, hat sich der Sauerstoffbedarf zusätzlich als nachteilig erwiesen. Vor allem dann, wenn viel Sauerstoff zur Oxidation hoher Konzentrationen an Enzymsubstrat gebraucht wird, kann die Diffusion von Sauerstoff aus der Luft in das Reaktionsmedium zum geschwindigkeitsbestimmenden Schritt werden und zu langen Reaktionszeiten oder insbesondere bei kinetischen Bestimmungsmethoden zu falschen Ergebnissen führen.

In US-A-4105800 wird Xanthin durch Oxidation mit Xanthinoxidase in Gegenwart von Resazurin, einem aromatischen N-Oxid, als Elektronenakzeptor durch Farbbildung nachgewiesen.

Dehydrogenasen können ganz allgemein in solche unterteilt werden, die für die Oxidation von Enzymsubstraten Nikotinamid-adenindinucleotid (NAD) oder Nikotinamid-adenin-dinucleotid-phosphat (NADP) als natürlichen unmittelbaren Elektronenakzeptor benötigen und in solche, die nicht NAD- oder NADP-abhängig sind und die also andere Substanzen als natürliche unmittelbare Elektronenakzeptoren bei enzymatischen Oxidationsreaktionen verwenden.

Der Einsatz von Dehydrogenasen für kolorimetrische Messungen ist z. B. aus DE-A-21 47 466 bekannt. Dort wird beschrieben, daß Lactat unter Katalyse von Lactat-Dehydrogenase mit Nikotinamidadenin-dinucleotid zu Pyruvat und reduziertem Nikotinamid-adenindinucleotid umgesetzt wird. Das gebildete NADH reagiert dann beispielsweise in Gegenwart des Enzyms Diaphorase mit Tetrazoliumsalzen unter Bildung von NAD und farbigen Formazanen, deren Konzentration photometrisch bestimmt werden kann. Anstatt Diaphorase ist auch N-Methylphenazinium-methosulfat als Reduktionskatalysator für die Übertragung der Elektronen von NADH auf das Tetrazoliumsalz genannt.

Nachteile dieses Verfahrens sind darin zu sehen, daß statt NADH auch andere eventuelle in biologischen Proben, wie z. B. Blut, Serum, Plasma oder Urin vorkommende, reduzierend wirkende Substanzen, wie Glutathion oder Medikamente, wie Methyldopa oder Dobesylat in Gegenwart von Reduktionskatalysatoren, wie Diaphorase oder N-Methylphenazinium-methosulfat Tetrazoliumsalze in entsprechende Formazane überführen und so zu falsch-positiven Ergebnissen führen.

Die Notwendigkeit von Reduktionskatalysatoren, wie Diaphorase oder Phenaziniumethosulfat für die Übertragung der durch die Oxidation eines Analyten freiwerdenden Elektronen vom Analyt/Enzymsystem auf einen der Farbbildung dienenden Elektronenakzeptor birgt außerdem den Nachteil in sich, daß dadurch auch andere Probenbegleitstoffe reduziert werden können, die unter sonst gleichen Bedingungen keine Reduktion eingingen, so aber zu falsch-negativen Resultaten führen.

In EP-A-0 120 440 wird Glycerin durch eine NAD-unabhängige Dehydrogenase in Gegenwart verschiedener Elektronenakzeptoren nachgewiesen. Insbesondere wird auch hier Phenazinmethosulfat verwendet, bevorzugt als Elektronenübertragungsreagenz auf einen weiteren Elektronenakzeptor wie z.B. ein Tetrazoliumsalz.

Den vorgenannten Nachteilen des Standes der Technik will die Erfindung Abhilfe schaffen. Der Erfindung lag speziell die Aufgabe zugrunde, fur die Analytik insbesondere von biologischen Flussigkeiten ein Verfahren zur kolorimetrischen Bestimmung eines Analyten mittels enzymatischer Oxidation des Analyten in Gegenwart eines Elektronenakzeptors und Bestimmung des Elektronenakzeptors durch Farbbildung als Maß fur die Menge des Analyten bereitzusteilen, bei dem reduzierend wirkende, insbesondere Wasserstoffperoxid zersetzende Begleitstoffe in Proben insbesondere biologischen Proben, wie von Blut abgeleiteten Flussigkeiten, z. B. Serum oder in Urin nicht storen, die Verwendung von Reduktionskatalysatoren, wie Diaphorase oder N-Methylphenazinium-methosulfat vermieden, fur das kein Sauerstoff erforderlich ist und das dadurch auch bei hohen Substratkonzentrationen schnelle Farb-Endwerte ermoglicht.

Außerdem sollte ein Mittel zur kolorimetrischen Bestimmung eines Analyten durch enzymatische Oxidation des Analyten, enthaltend eine Oxidoreduktase und einen farbbildenden Elektronenakzeptor zur Verfügung gestellt werden, welches für die Durchfuhrung des vorstehend genannten Verfahrens geeignet ist.

Im einzelnen erstreckt sich die Aufgabe auf die Auswahl solcher Substanzen, die als farbbildende Elektronenakzeptoren für ein verbessertes Verfahren und Mittel wie oben angegeben eingesetzt werden können.

Die Aufgabe wird durch die Erfindung, wie sie in den Anspruchen charakterisiert ist, gelöst.

Demgemäß wurde ein Verfahren zur kolorimetrischen Bestimmung eines Analyten mittels enzymatischer Oxidation des Analyten in Gegenwart eines Elektronenakzeptors und Bestimmung des reduzierten Elektronenakzeptors durch Farbbildung als Maß für die Menge des Analyten gefunden, das dadurch gekennzeichnet ist, daß der Analyt

mit einer flavinabhängigen Oxidase oder einer PQQ-abhängigen Dehydrogenase in Anwesenheit einer aromatischen Nitrosoverbindung oder eines tautomeren äquivalenten Oxims als direktem Elektronenakzeptor oxidiert wird.

Außerdem wurde ein Mittel zur kolorimetrischen Bestimmung eines Analyten durch enzymatische Oxidation des Analyten gefunden, das eine flavinabhängige Oxidase oder eine PQQ-abhängige Dehydrogenase und einen farbbildenden Elektronenakzeptor enthält, das dadurch gekennzeichnet ist, daß der farbbildende Elektronenakzeptor eine direkt mit dem Analyt / Enzym-System reagierende aromatische Nitrosoverbindung ist.

Erfindungsgemäß wird unter "Analyt" eine solche Substanz verstanden, die enzymatisch oxidiert wird. In vielen Fällen wird der Analyt diejenige Substanz sein, die in der zu untersuchenden Probe direkt nachgewiesen oder quantitativ bestimmt werden soll. Beispielsweise kann Glucose direkt mit Glucoseoxidase oxidiert und kolorimetrisch bestimmt werden. Es ist Jedoch auch möglich, daß der Analyt erst durch eine oder mehrere vorgeschaltete Reaktionen aus einer anderen Substanz gebildet wird und so aus der Anwesenheit und Konzentration des Analyten indirekt auf die Anwesenheit und Konzentration der Ausgangssubstanz geschlossen werden kann. So kann z. B. Glycerin so nachgewiesen und bestimmt werden, daß in einer ersten Reaktion Glycerin mittels Glycerinkinase und Adenosintriphosphat in Glycerin-3-phosphat und Adenosin-diphosphat überführt wird und dann anschließend in einer zweiten Reaktion Glycerin-3-phosphat mit Glycerin-3-phosphat-oxidase oxidiert wird. Glycerin-3-phosphat ist in diesem Fall der Analyt, dessen Konzentration derjenigen der zu bestimmenden Substanz, nämlich Glycerin, entspricht. Der Analyt ist jedoch auch hier diejenige Verbindung, die kolorimetrisch bestimmt wird.

Der Analyt ist in der vorliegenden Erfindung diejenige Substanz, die als Substrat des jeweiligen oxidierenden Enzyms akzeptiert wird. Damit der Analyt oxidiert wird, muß gleichzeitig ein Elektronenakzeptor zugegen sein, der die Elektronen unter Mitwirkung des Enzyms von dem Analyten übernimmt.

Unter "farbbildend" wird verstanden, daß der Elektronenakzeptor nach Reduktion entweder unmittelbar selbst in einer anderen Farbe vorliegt als vor der enzymatischen Oxidation des Analyts oder daß der reduzierte Akzeptor selbst zwar nicht direkt zu einer Farberzeugung führt, er in einer Folgereaktion aber ursächlich zu einer Farbänderung der Reaktionsmischung führt. Farbänderung umfaßt hierbei sowohl den Wechsel von farblos nach farbig als auch von einer Farbe zu einer anderen. Für die Farbbildung durch Folgereaktionen sind dem Fachmann viele Möglichkeiten bekannt, aus denen er je nach den Gegebenheiten auswählen kann. Beispielsweise seien hier genannt solche Reaktionen, bei denen der reduzierte Elektronenakzeptor selbst Teil einer farbigen Verbindung wird, wie oxidative Kupplungsreaktionen. Farbbildende Folgereaktionen können aber auch solche sein, bei denen der reduzierte Elektronenakzeptor durch seine reduzierende Wirkung auf eine andere Substanz zu einer Farbänderung derselben führt.

Unter Oxidationsstufe wird ein Zahlenwert verstanden, der den Oxidationszustand eines bestimmten Atoms in einer Verbindung, die ein neutrales Molekül oder ein geladenes Komplexion sein kann, kennzeichnet. Dem Fachmann ist die Ermittlung von oxidationsstufen bekannt. Anleitungen zur Bestimmung dieses Zahlenwertes sind grundlegenden Lehrbüchern der Chemie, wie z. B. "Anorganische Chemie", Hofmann & Rudorff, Verlag F. Vieweg & Sohn, Braunschweig, 20. Auflage, 1969, Seite 216 - 218; "Lehrbuch der anorganischen Chemie", Hollemann-Wiberg, Verlag Walter deGruyter & Co., Berlin, 71. - 80. Auflage, 1971, Seite 197 - 199 oder "Anorganikum", Hrsg. L. Kolditz, VEB Deutscher Verlag der Wissenschaften, Berlin, 4. Auflage, 1972, Seite 446 zu entnehmen.

Geeignete Nitrosoverbindungen, die für das erfindungsgemäße Verfahren zur kolorimetrischen Bestimmung eines Analyten einsetzbar sind, sind vorzugsweise solche, die den Nitrosorest an eine aromatische Gruppe gebunden enthalten.

Besonders bevorzugt sind kohlenstoffaromatische Nitrosoverbindungen, wie Nitrosobenzol und Nitrosobenzol-Derivate. Als Nitrosobenzol-Derivate sind hierbei alle diejenigen für das erfindungsgemäße Verfahren ganz hervorragend geeignet, die durch Reduktion in solche Verbindungen überführt werden, die zur Farbbildung im Sinne der zuvor gegebenen Erläuterung dienen können.

Nitrosobenzol und Nitrosobenzol-Derivate sind in der Regel schwach farbige, gelb, grün oder braun gefärbte Substanzen, die im reduzierten Zustand nicht gefärbt sind. Sie müssen deshalb, um einen Analyten kolorimetrisch nachweisen zu können, in einer Folgereaktion zu einer Farbbildung führen. Dies kann beispielsweise dadurch erreicht werden, daß die reduzierte Nitrosoverbindung mit einer anderen Substanz so umgesetzt wird, daß eine farbige Substanz erhalten wird, die die reduzierte Nitrosoverbindung als Teilstruktur enthält.

Oxidative Kupplungsreaktionen sind eine Möglichkeit für diese Art der Farbbildung. Deswegen sind insbesondere solche Nitrosobenzol-Derivate für das erfindungsgemäße Verfahren geeignet, die nicht nur als Elektronenakzeptoren für oxidierende Enzyme wirken, sondern die auch im reduzierten Zustand für oxidative Kupplungsreaktionen einsetzbar sind.

Ganz besonders bevorzugt sind Nitrosobenzol-Derivate der allgemeinen Formel II 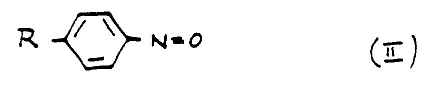
in der
- R: eine Hydroxygruppe oder eine Aminogruppe bedeuten,
wobei die Aminogruppe ein- oder zweifach durch Niederalkyl substituiert sein und die Niederalkylgruppe ihrerseits durch eine Hydroxygruppe, eine ein- oder mehrfach durch Niederalkyl substituierte Aminogruppe, PO₃H₂, SO₃H oder CO₂H substituiert sein kann.

Hervorragend geeignet sind insbesondere solche Nitrosobenzol-Derivate der allgemeinen Formel II, die schwer flüchtig sind.

Niederalkyl bedeutet in vorstehender Definition einen Alkylrest mit 1 - 5 Kohlenstoffatomen. Besonders bevorzugt sind Methyl und Ethyl.

Die Säurereste PO₃H₂, SO₃H und CO₂H können als solche oder in Salzform als Ammonium-, Alkali- oder Erdalkalisalze vorliegen. Ammoniumsalze sind solche, die das unsubstituierte Ammoniumkation NH₄⁺ enthalten oder solche, die ein- oder mehrfach durch Niederalkyl, Aryl oder/und Niederalkylaryl substituierte Ammoniumkationen enthalten. Niederalkyl bedeutet jeweils ein 1 - 5 Kohlenstoffatome enthaltender Alkylrest. Der Arylrest ist ein 6 - 10 Kohlenstoffatome zählendes aromatisches Ringsystem.

Als Niederalkyl werden Methyl und Ethyl bevorzugt. Bevorzugter Arylrest ist Phenyl, wobei Benzyl ein bevorzugter Niederalkylarylrest ist.

Als Elektronenakzeptoren für das erfindungsgemäße Verfahren eignen sich p-Hydroxy-nitrosobenzol, p-Dimethylamino-nitrosobenzol, p-Diethylamino-nitrosobenzol und p-Dihydroxyethylamino-nitrosobenzol ganz hervorragend.

Wie vorstehend ausgeführt, werden für das erfindungsgemäße Verfahren bevorzugt Nitrosobenzol oder Nitrosobenzol-Derivate, in denen Stickstoff in der Oxidationsstufe + 1 vorliegt, mit der zu untersuchenden Probe und einem oxidierenden Enzym in Kontakt gebracht. Bei Anwesenheit eines Analyten in der Probe, der als Enzymsubstrat akzeptiert wird, fungiert Nitrosobenzol oder ein Nitrosobenzolderivat als Elektronenakzeptor und wird dadurch zum entsprechenden Amin reduziert. In der Regel reicht diese Reduktion der Nitrosoverbindungen allein nicht für eine kolorimetrische Bestimmung des Analyten aus, da entweder nur Farbabnahmen auftreten oder Farbänderungen nur schwach wahrnehmbar sind. Der reduzierte Elektronenakzeptor kann aber als Ausgangsmaterial für eine oxidative Kupplungsreaktion dienen, wobei je nach Wahl des Kupplungspartners die unterschiedlichsten Farben erhalten werden können.

Oxidative Kupplungsreaktionen sind dem Fachmann bekannt und beispielsweise in EP-A-0 175 250 oder in H. Hünig et al., Angewandte Chemie 70, 215 - 222 (1958) beschrieben. Allgemein werden oxidative Kupplungen häufig zur Herstellung von Farbstoffen angewandt. Sie können in diesem Zusammenhang als Reaktion zwischen einer elektronenreichen aromatischen Verbindung und einer oxidablen Kupplungskomponente aufgefaßt werden, die in Gegenwart von Oxidationsmitteln, wie z. B. Natrium- oder Kaliumhexacyanoferrat(III), Kupfersalzen, Quecksilbersalzen, Eisen(III)chlorid, Bleidioxid, Wasserstoffperoxid, Bleitetraacetat, Natrium- oder Kaliumsalzen der Perschwefelsäure, Peressigsäure, Perjodsäure oder Chloramin T ablaufen. Gemäß DE-A 33 31 588 lassen sich auch Oxidasen als Oxidationsmittel verwenden. Die vorstehend beschriebenen Nitrosobenzole stellen in ihrem reduzierten Zustand, nachdem sie als Elektronenakzeptoren in enzymatischen Oxidationen gewirkt haben, Verbindungen dar, die als oxidable Kupplungskomponenten für oxidative Kupplungsreaktionen zur Verfügung stehen. Hierfür können die durch die Reduktion entstandenen Amine durch Oxidationsmittel, wie sie vorstehend beispielhaft genannt sind, oxidiert werden, um mit gleichzeitig in der Reaktionsmischung anwesenden elektronenreichen Kupplungskomponenten einen Farbstoff zu bilden.

Dem Fachmann stehen eine Vielzahl elektronenreicher Kupplungskomponenten zur Verfügung. Diese Kupplungskomponenten sind je nach der gewünschten Farbe des Kupplungsproduktes wählbar. Beispiele sind aromatische Amine, Phenole oder methylenaktive Verbindungen. Besonders bevorzugte Verbindungen können aus der Gruppe der Aniline, z. B. N-Methylanthranilsäure und den in EP-A-0 175 250 genannten Anilinophosphonsäuren gewählt werden.

Eine weitere Möglichkeit zur Bestimmung des reduzierten Elektronenakzeptors durch Farbbildung besteht im Falle der Nitrosoverbindungen darin, die reduzierte Nitrosoverbindungen durch andere Substanzen zu oxidieren, die ihrerseits aufgrund dieses Übergangs von der oxidierten in die reduzierte Form eine Farbe bilden, wobei auch hier unter Farbbildung sowohl die Übergänge von einer Farbe in die andere ebenso wie vom farblosen zum farbigen Zustand verstanden werden sollen. Beispiele für solche Farbbildungsreaktionen sind z. B. die Oxidation des reduzierten Elektronenakzeptors mittels Metallsalzen, die hierbei zu farbigen Metallsalzen mit dem Metall in einer niedrigeren Oxidationsstufe oder eventuell ganz bis zum neutralen Metall reduziert werden. Kupfer(II)-salze können beispielsweise in rote Kupfer(I)-salze und Silbersalze in metallisches Silber überführt werden. Möglich ist auch die Reduktion von Phosphormolybdat zu Molybdänblau.

Anstelle von aromatischen Nitrosoverbindungen können für das erfindungsgemäße Verfahren auch aromatische Oxime als Elektronenakzeptoren für enzymatische Oxidation eingesetzt werden. Nitrosoverbindungen können mit Oximen in einem tautomeren Gleichgewicht stehen. Dies ist insbesondere der Fall, wenn hierfür ein Wasserstoffatom in α-Position zur Nitrosogruppe zur Verfügung steht 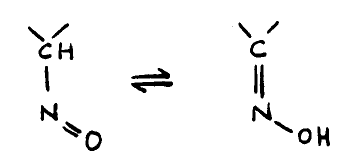
oder wenn eine entsprechende Delokalisation der Elektronen und Protonen möglich ist.

Es wurde gefunden, daß Oxime, die mit Nitrosoverbindungen, welche als Elektronenakzeptoren in enzymatischen Oxidationsreaktionen funktionieren, in einem tautomeren Nitroso/Oxim-Gleichgewicht stehen können, ebenfalls in erfindungsgemäßen Verfahren verwendet werden können. Insbesondere trifft dies zu für Oxime der allgemeinen Formel III 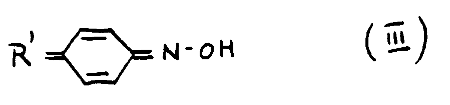
in der
- R': Sauerstoff, eine weitere Oximgruppe oder eine positiv geladene Aminogruppe bedeuten, wobei die Aminogruppe ein- oder zweifach durch Niederalkyl substituiert sein und die Niederalkylgruppe ihrerseits durch eine Hydroxygruppe eine ein- oder mehrfach durch Niederalkyl substituierte Aminogruppe, PO₃H₂, CO₂H oder SO₃H substituiert sein kann.

Niederalkyl bedeutet in vorstehender Definition einen Alkylrest mit 1 - 5 Kohlenstoffatomen. Besonders bevorzugt sind Methyl und Ethyl.

Die Säurereste PO₃H₂, SO₃H und CO₂H können als solche oder in Salzform als Ammonium-, Alkali- oder Erdalkalisalze vorliegen.

Ammoniumsalze sind solche, die das unsubstituierte Ammoniumkation NH₄⁺ enthalten oder solche, die ein- oder mehrfach durch Niederalkyl, Aryl oder/und Niederalkylaryl substituierte Ammoniumkationen enthalten. Niederalkyl bedeutet jeweils einen 1 -5 Kohlenstoffatome enthaltenden Alkylrest. Der Arylrest ist ein 6 - 10 Kohlenstoffatome zählendes aromatisches Ringsystem.

Als Niederalkyl werden Methyl und Ethyl bevorzugt. Bevorzugter Arylrest ist Phenyl, wobei Benzyl ein bevorzugter Niederalkylarylrest ist.

In solchen Oximen besitzt Stickstoff die Oxidationsstufe - 1.

Ebenso wie Nitrosobenzol sind auch die Oxime der allgemeinen Formel III farblose oder gefärbte Substanzen, die im reduzierten Zustand nicht gefärbt sind. Wie vorstehend für Nitrosobenzole als Elektronenakzeptoren im erfindungsgemaßen Verfahren beschrieben, können auch aromatische Oxime nach ihrer Funktion als Elektronenakzeptoren zur Farbbildung gebracht werden. Sowohl Nitrosoverbindungen wie auch Oxime ergeben bei erschöpfender Reduktion Amine. Für Oxime als Elektronenakzeptoren sind in dem erfindungsgemäßen Verfahren grundsätzlich die gleichen Folgereaktionen zur Farbbildung anwendbar wie für Nitrosoverbindungen.

Überraschenderweise wurde gefunden, daß Nitrosoverbindungen und Oxime, wie sie vorstehend beschrieben sind, als farbbildende Elektronenakzeptoren für viele enzymatische Oxidationen dienen können, die durch Oxidoreduktasen katalysiert werden. Bevorzugt sind sie dort einsetzbar, wo für die Elektronenübertragung von zu oxidierenden Analyten auf den farbbildenden Elektronenakzeptor keine Reduktionskatalysatoren, wie Diaphorase oder N-Methylphenazinium-methosulfat gewünscht werden. Überraschenderweise wurde gefunden, daß diese Substanzen vorteilhaft sind, wenn Oxidasen oder PQQ-abhängige Dehydrogenasen als oxidierende Enzyme eingesetzt werden. Werden solche Enzyme für die Oxidation eines Analyten verwendet, kann die Elektronenübertragung direkt, d. h. ohne Mitwirkung eines Reduktionskatalysators von dem Analyt/Enzym-System auf die vorgenannten farbbildenden Elektronenakzeptoren erfolgen. Als Analyt/Enzym-System wird in diesem Zusammenhang die für die Oxidationsreaktion notwendige Kombination aus Analyt und oxidierendem Enzym sowie gegebenenfalls zusammen mit fur die Oxidation naturlicherweise notwendigen mit dem Enzym zusammenwirkenden Coenzymen und oder Cofaktoren, wie beispielsweise Metallsalzen, verstanden.

Fur das erfindungsgemaße Verfahren sind flavinabhangige Oxidasen besonders bevorzugt. Beispiele sind L- und D-Aminosaure-Oxidasen, Cholesterin-Oxidase, Glucose-Oxidase, Glycerin-3-phosphat-Oxidase, Lactat-Oxidase und Pyruvat-Oxidase. Oxidasen, die erfindungsgemaß ganz besonders geeignet sind, sind Glucose-Oxidase, Glycerin-3-phosphat-Oxidase, Lactat-Oxidase und Pyruvat-Oxidase.

Weiter sind Pyrrolo-chinolin-chinon (PQQ)-abhängige Dehydrogenasen für das erfindungsgemäße Verfahren besonders vorteilhaft einsetzbar. Insbesondere Glucose-Dehydrogenase eignet sich gut zur kolorimetrischen Bestimmung von Glucose bei Anwesenheit von aromatischen Nitrosoverbindung als farbbildenden Elektronenakzeptoren. Ebenso kann PQQ-abhängige Alkohol-Dehydogenase zur Bestimmung von Alkohol, wie Ethanol, eingesetzt werden.

Das erfindungsgemäße Verfahren wird so durchgeführt, daß die zu untersuchende Probe mit einer geeigneten Oxidoreduktase und einem oder mehreren der vorstehend beschriebenen farbbildenden Elektronenakzeptoren kontaktiert wird. Enthalt die Probe einen Analyten, der von der Oxidoreduktase oxidiert wird, wird der farbbildende Elektronenakzeptor reduziert. Weist der reduzierte Elektronenakzeptor eine andere Farbe auf als der ursprüngliche Elektronenakzeptor in seiner oxidierten Form oder ging der Elektronenakzeptor durch den enzymatischen Oxidationsvorgang vom farblosen Zustand in einen gefärbten über, kann die Intensität der gebildeten Farbe direkt visuell, eventuell durch Vergleichsfarben oder photometrisch mit der Konzentration des Analyten in der Probe korreliert werden. Ist die Farbe des reduzierten Elektronenakzeptors im wesentlichen die gleiche wie die des ursprünglich eingesetzten Elektronenakzeptors oder findet eine Farbbleiche oder völlige Entfärbung statt, so wird eine Folgereaktion an die enzymatische Oxidationsreaktion angeschlossen, die zu einer Farbe führt, aus deren Intensität visuell oder photometrisch ebenfalls die Konzentration des Analyten in der Probe ermittelt werden kann.

Das Verfahren kann in einem sogenannten Naßtest, z. B. in einer Küvette oder als sogenannter Trockentest auf einem entsprechenden Reagenzträger durchgeführt werden, wobei die notwendigen Testreagenzien auf einem festen Träger, insbesondere einem saugfähigen oder quellbaren Material vorliegen. Solche Testträger sind z. B. aus EP-A-0 016 387, DE-A-32 47 608, EP-A-0 262 445 oder EP-A-0 256 806 bekannt.

Mittel zur kolorimetrischen Bestimmung eines Analyten zur Durchführung des erfindungsgemäßen Verfahrens, wie sie in den Ansprüchen gekennzeichnet sind, sind ebenfalls Gegenstand der Erfindung. Ein solches Mittel enthält neben der für die enzymatische Oxidation des zu bestimmenden Analyten notwendigen Oxidoreduktase mindestens einen farbbildenden Elektronenakzeptor, der die bei der Oxidation freiwerdenden Elektronen direkt von dem Analyt/Enzym-System übernimmt. Als oxidierende Enzyme und farbbildende Elektronenakzeptoren werden die vorstehend für das erfindungsgemäße Verfahren beschriebenen Stoffe eingesetzt.

Zur Einhaltung eines für die Durchführung des Verfahrens geeigneten pH-Wertes, der sich insbesondere nach den einzusetzenden Enzymen richtet, enthält das erfindungsgemäße Mittel ein Puffersystem. Außerdem kann es weitere geeignete üblicherweise für solche Mittel verwendete Zusatzstoffe, wie beispielsweise Netzmittel, Stabilisatoren etc. enthalten. Führt die Oxidation des Elektronenakzeptors nicht zu einer meßbaren Farbänderung, schließt das erfindungsgemäße Mittel zur kolorimetrischen Bestimmung eines Analyten natürlich auch die für eine Folgereaktion erforderlichen Reagenzien ein.

Das erfindungsgemäße Mittel kann in Form einer Lösung oder auf einen saugfähigen oder quellbaren Träger aufgezogen vorliegen. In Form einer Lösung enthält das Mittel vorzugsweise sämtliche für das erfindungsgemäße Verfahren benötigten Reagenzien. Als Lösungsmittel kommen Wasser, wasserlösliche organische Lösungsmittel, wie z. B. Methanol, Ethanol, Aceton oder Dimethylformamid oder Mischungen aus Wasser mit solchen organischen Lösungsmitteln in Frage. Aus Haltbarkeitsgründen kann es vorteilhaft sein, die für den Test benötigten Reagenzien auf zwei oder mehr Lösungen zu verteilen, die erst bei der eigentlichen Untersuchung vermischt werden. Letzteres kann insbesondere dann zutreffen, wenn nach Oxidation des Analyten der reduzierte Elektronenakzeptor in einer Folgereaktion, z. B. einer oxidativen Kupplungsreaktion weiter umgesetzt wird. Typische Konzentrationen für die im erfindungsgemäßen Mittel eingesetzten Elektronenakzeptoren sind: 0,01 - 100 mMol/l, bevorzugt 0,1 - 25 mMol/l. Reagenzien für Folgereaktionen werden mindestens im stöchiometrischen Verhältnis zu den Elektronenakzeptoren eingesetzt, bevorzugt in einem Überschuß, insbesondere in einem 2 - 10fachen Überschuß.

Das erfindungsgemäße Mittel kann auch in Form eines Teststreifens vorliegen. Solche Teststreifen sind in vielen Ausführungsarten bekannt, beispielsweise aus EP-A-0 016 387, DE-A-32 47 608, EP-A-0 262 445 oder EP-A-0 256 806. Allen gemeinsam ist, daß die zur Durchführung des Bestimmungsverfahrens benötigten Reagenzien auf festen Trägerschichten vorliegen. Als Trägerschichten kommen insbesondere saugfähige und/oder quellbare Materialien in Frage, die von der zu untersuchenden Probenflüssigkeit benetzt werden. Beispiele sind Gelatine, Zellulose oder Kunstfaservliese. In oder auf diesen Trägermaterialien liegen die Reagenzien in fester Form vor.

Bei Aufgabe der Probenflüssigkeit auf den Teststreifen oder Eintauchen des Teststreifens in die Probenflüssigkeit bildet sich in dem Streifen ein flüssiges Milieu aus, innerhalb dessen die Nachweisreaktion abläuft. Die durch die Reaktion verursachte Farbbildung kann visuell oder photometrisch z. B. reflexionsphotometrisch ausgewertet werden.

Ein spezieller Gegenstand der Erfindung betrifft die Verwendung einer aromatischen Nitrosoverbindung als direkten Elektronenakzeptor eines Analyt / Enzym-Systems. Als Analyt / Enzym-System wird in diesem Zusammenhang die für eine enzymatische Oxidationsreaktion notwendige Kombination aus Analyt und flavinabhängiger Oxidase oder PQQ-abhängiger Dehydrogenase sowie gegebenenfalls zusammen mit für die Oxidation naturlicherweise notwendigen, mit dem Enzym zusammenwirkenden Coenzymen wie Flavin oder PQQ oder / und Cofaktoren, wie beispielsweise Metallsalzen, verstanden. Es wurde gefunden, daß Substanzen aus der Gruppe der Nitrosoverbindungen und Oxime ganz allgemein als farbbildende Elektronenakzeptoren bei der Oxidation eines Analyten mittels einer Oxidoreduktase eingesetzt werden können.

Die vorliegende Erfindung bietet den Vorteil, daß keine Reduktionskatalysatoren, wie Diaphorase oder N-Methylphenaziniummethosulphat zur Reduktion eines farbbildenden Elektronenakzeptors notwendig sind. Dessen Reduktion kann nun direkt durch das Analyt/Enzym-System erfolgen. Eventuelle storende Nebenreaktionen konnen so vermieden werden.

Insbesondere im Falle des Einsatzes von Oxidasen zur enzymatischen Oxidation von Analyten wird durch Einsatz der erfindungsgemaßen Verbindungen die Bildung von Wasserstoffperoxid als Vorstufe eines kolorimetrischen Bestimmungsverfahrens vermieden. Dies beseitigt oder vermindert den störenden Einfluß reduzierend wirkender Verbindungen.

Schließlich bieten die erfindungsgemaß angewendeten Verbindungen eine echte Alternative uberall dort, wo der Zutritt von Luftsauerstoff limitiert oder unerwunscht ist. Sauerstoff kann als Elektronenakzeptor bei enzymatischei Oxidationen durch diese Verbindungen ersetzt werden. Ganz besondere Vorteile bietet dies bei erfindungsgemaßen Mitteln in Form von Teststreifen. Während diese bisher besonders bei hohen Analytkonzentrationen so aufgebaut sein mußten, daß bei enzymatischen Oxidationen mittels Oxidase Luftsauerstoff Zutritt zu der auf den Teststreifen aufgebrachten Reagenzmischung hat, können nun Teststreifen konstruiert werden, die besonders schnell und zuverlässig arbeiten. Während so bisher bei Filmteststreifen oft die Probe nach Aufgabe auf den Teststreifen nach einer bestimmten Zeit wieder abgewischt werden mußte, damit Sauerstoff überhaupt in den Teststreifen eindiffundieren konnte, ist diese Maßnahme bei Benutzung der erfindungsgemäßen Elektronenakzeptoren nicht nötig. Dadurch, daß die zeitabhängige Diffusion von Sauerstoff in den Teststreifen vermieden wird, werden bei kinetischen Messungen von der Analytkönzentration abhängige Reaktionsgeschwindigkeiten und bei Endpunktmessungen im Endpunkt von der Analytkonzentration unabhängige Reaktionsgeschwindigkeiten erhalten, die schnellere, zuverlässigere und einfachere Bestimmungsverfahren erlauben, als es bisher möglich war.

Die nachfolgenden Beispiele zeigen einige der möglichen Verfahrensvarianten zur kolorimetrischen Bestimmung eines Analyten mittels enzymatischer Oxidation.

### Beispiel 1

### Reduktion von Nitrosoverbindungen durch Glucose-Oxidase und Glucose

A) 2 000 »l 0,1 M Citronensaure/NaOH-Puffer, pH 6,0
   200 »l p-Nitroso-N,N-dimethylanilin, 0,1 M in Ethanol
   100 »l Glucose-Oxidase (EC 1.1.3.4) (2500 IU/ml) oder Glucose-Dehydrogenase (EC1.1.99.10)
   200 »l Probe mit bekanntem Glucosegehalt
   a) 5 mM
   b) 10 mM
   c) 15 mM
   d) 20 mM
   e) 50 mM
      in Wasser
   wurden gemischt und 2 Minuten bei 25 °C inkubiert. Dann wurden
   250 »l N-Methylanthranilsäure, 0,1 M in Ethanol,
   125 »l Kaliumhexacyanoferrat(II), 0,2 M in Wasser und
   125 »l Kaliumhexacyanoferrat(III), 0,2 M in Wasser
   zugesetzt. Nach einer weiteren Minute wurde 25fach verdünnt und die Absorption der Reaktionsmischung, die sich bei Anwesenheit von Glucose grün farbt, bei 710 nm gegen einen Blindwert (vorstehende Reaktionsmischung ohne Glucose) gemessen. Die erhaltenen Resultate ergaben eine Gerade. Der Extinktionskoeffizient von ε₇₁₀ = 24 000 M⁻¹ cm⁻¹ kann zur Bestimmung von unbekannten Glucosekonzentrationen in Lösungen herangezogen werden.
B) Wurde bei vorstehendem Test unter A) p-Nitroso-dimethylanilin ersetzt durch
   a) p-Nitroso-phenol
   b) p-Nitroso-N,N-diethylanilin
   c) p-Nitroso-N,N-diethanolanilin (Herstellung nach D'Amico et al., J. Amer. Chem. Soc. 81, 5957 (1959))
   so wurde bei Anwesenheit von Glucose durch die Kupplung mit N-Methylanthranilsäure ein Farbwechsel von
   a) braun nach blau
   b) gelb nach grün
   c) gelb nach grün
   beobachtet.
C) Wurde bei vorstehendem Test unter A) N-Methylanthranilsäure ersetzt durch
   a) N-Methyl-N-methylenphosphonsäure-anilin
   b) 1-Hydroxy-naphthalin-2-carbonsäure
   c) Anilin-2-sulfonsäure
   so wurde bei Anwesenheit von Glucose durch die Kupplung mit p-Nitroso-N,N-dimethylanilin eine Farbe mit
   a) λₘₐₓ = 735 nm
   b) λₘₐₓ = 590 nm
   c) λₘₐₓ = 640 nm
   beobachtet.

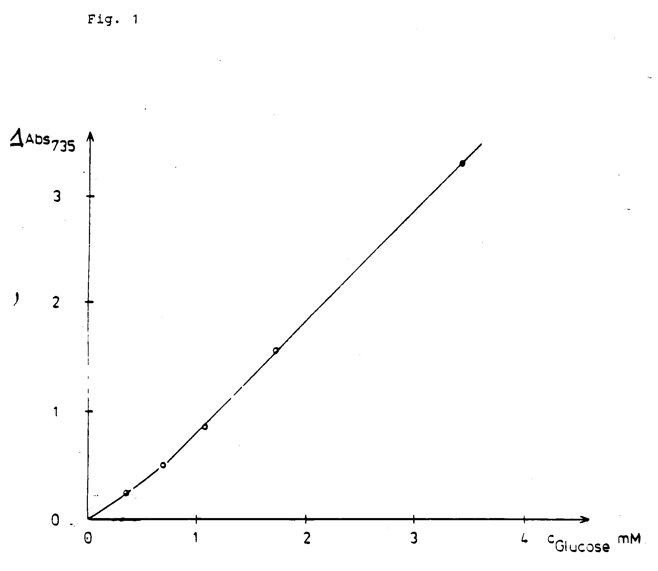

Im Falle des Einsatzes von p-Nitroso-N,N-dimethylanilin und N-Methyl-N-methylenphosphonsäure-anilin wurde die in Fig. 1 wiedergegebene Abhängigkeit der Änderung der Lichtabsorption bei 735 nm von der Glucose-Konzentration gefunden. Hierzu wurde die Extinktion nach (1+24)-Verdünnung in Citratpuffer, pH 6 gemessen und gegen die Glucosekonzentration im Testansatz aufgetragen.

### Beispiel 2

### Nachweis von Glucose durch Bildung metallischen Silbers

Der Ansatz aus Citratpuffer, p-Nitroso-N,N-dimethylanilin, Glucose-Oxidase und Probe wie in Beispiel 2 A wurde 2 Minuten bei 25 °C inkubiert und mit 250 »l 100 mM Silbernitrat-Losung in Wasser sowie 250 »l Goldsol in Wasser versetzt.

(Die Herstellung von Goldsol erfolgte nach folgender Vorschrift: Zu 100 ml kochendem destilliertem Wasser werden nacheinander 0,4 mg HAuCl₄ in 0,4 ml Wasser; 0,2 ml 0,1 M NaSCN in Wasser und 0,5 ml 0,1 M K₂CO₃ in Wasser gegeben. Nach 10 Minuten wird abkuhlen gelassen.)

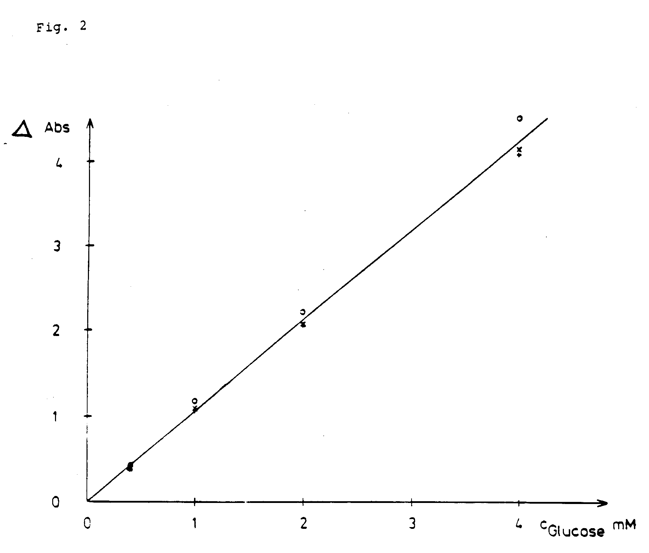

Mit den ohne Zwischenverdünnung erhaltenen Resultaten bei 700, 850 und 1300 nm wurden die in Figur 2 wiedergegebenen Kurven erhalten. Sie können als Eichkurven zur Ermittlung des unbekannten Glucosegehalts in Lösungen dienen.

### Beispiel 3

### Nachweis von Glucose durch Molybdänblau-Bildung

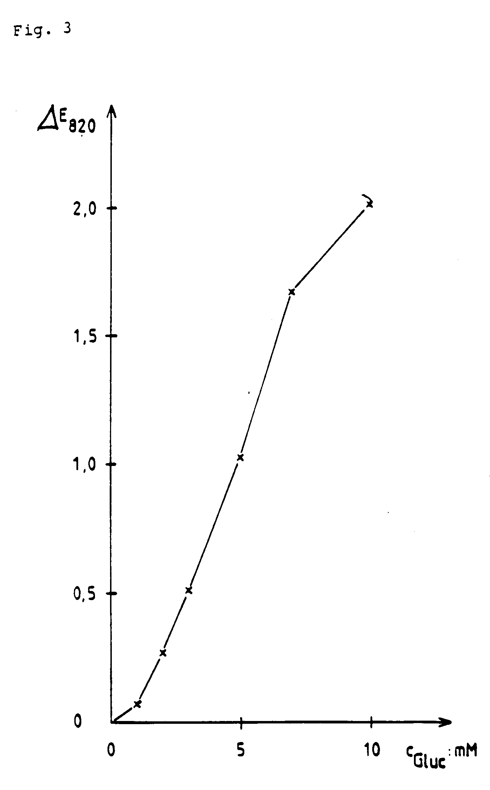

Zu einer Lösung von 200 mg 2,18-Phosphormolybdänsäure (Herstellung z. B. möglich nach G. Brauer, "Handbuch der präparativen Anorganischen Chemie", Enke-Verlag, Stuttgart, S. 1278 (1954) oder A. Rosenheim, A. Traube, Z. Anorg. Chemie 65, 99 (1910)) in 920 -x »l 0,1 M Citronensäure/NaOH-Puffer pH 5,5 wurden 40 »l 0,1 M p-Nitroso-N,N-dimethylanilin (in Ethanol) sowie 40 »l Glucose-Oxidase (6250 IU/ml Wasser) zugesetzt. 1 Minute nach Zugabe von x »l (x = 0, 1, 2, 3, 5, 7, 10) einer glucosehaltigen Probe bekannten Glucosegehaltes (1M) wurde auf 50 ml verdünnt und die Absorptionsänderung (ΔE) bei 820 nm gemessen. Als Resultat wurde die in Fig. 3 wiedergegebene Kurve erhalten. Sie kann als Kurve zur Ermittlung des unbekannten Glucosegehalts von Lösungen dienen, wobei C die Konzentration in der Probe vor der zur Messung durchgeführten Verdünnung darstellt.

### Beispiel 4

### Kinetische Bestimmung von Glucose mittels nicht-NAD(P)-abhängiger Glucosedehydrogenase

Es wurden folgende Lösungen hergestellt:
- Testpuffer:: 0,1 M Tris/Salzsaure, pH 7,5 enthaltend 1 % Rinderserumalbumin
- Elektronenakzeptor:: 0,1 M p-Nitroso-N,N-dimethylanilin in Ethanol
- Indikator:: 2,18-phosphormolybdänsäure, 100 mg/ml Wasser
- Enzym:: Glucose-Dehydrogenase (EC 1.1.99.17), 50 IU/ml Testpuffer
- Glucose-Lösung:: a) 36 mg Glucose/dl Humanplasma
b) 72 mg Glucose/dl Humanplasma
c) 144 mg Glucose/dl Humanplasma
d) 360 mg Glucose/dl Humanplasma
e) 720 mg Glucose/dl Humanplasma
f) 1440 mg Glucose/dl Humanplasma
g) 3600 mg Glucose/dl Humanplasma

In eine 1 cm-Küvette wurden 1740 »l Puffer
250 »l Elektronenakzeptor
250 »l Indikator und
10 »l Glucose-Dehydrogenase gegeben, die Mischung auf 25 °C thermostatiert und dann 250 »l Glucose-Lösung zugegeben. Mit der Zugabe der Glucose-Lösung als Startpunkt wurde die Absorptionsänderung pro Minute (ΔE/min) bei 820 nm registriert. Es wurden die folgenden Werte erhalten:

| Glucose-Konzentration (Endkonzentration im Test-Ansatz) | ΔE/min |
|---|---|
| 3,6 mg/dl | 0,20 |
| 7,2 mg/dl | 0,38 |
| 14,4 mg/dl | 0,57 |
| 36 mg/dl | 1,00 |
| 72 mg/dl | 1,60 |
| 144 mg/dl | 2,24 |
| 360 mg/dl | 3,13 |

### Beispiel 5

### Teststreifen zum Glucosenachweis durch Molybdanblau-Bildung

- 1 g: Natrium-Alginat (z. B. Algipon von Kelco, Division of Merck & Co., Clark, New Jersey, USA)
- 45 g: Polyvinylpropionat (z. B. Propiophan 70D von BASF, Ludwigshafen, Bundesrepublik Deutschland)
- 0,75 g: Natrium-nonylsulfat
- 10,15 g: Kalium-dihydrogenphosphat und
- 58,5 g: destilliertes Wasser
- 4 g: Aerosil COK 84 (Degussa, Hanau, Deutschland/BRD)

werden zu einer homogenen Massc verrührt und mit 10 N Natronlauge auf pH 5,5 eingestellt.

Anschließend wurden dann
65 mg Glucose-Oxidase (250 IU/mg),
260 mg p-Nitroso-N,N-dimethylanilin
1,3 g 2,18-Phosphormolybdänsäure
zugesetzt.

Die Masse wurde in 320 »m Schichtdicke auf eine 1 mm dicke Polystyrolfolie aufgerakelt und 1 Stunde bei 60 °C getrocknet. Bei Aufgabe eines Tropfens einer glucosehaltigen Lösung tritt innerhalb einer Minute eine deutliche Grünfärbung auf. Die Intensität der Färbung verstärkt sich mit zunehmender Glucosekonzentration und kann visuell anhand einer Vergleichsskala oder reflexionsphotometrisch ausgewertet werden.

Statt des Elektronenakzeptors p-Nitroso-N,N-dimethylanilin konnte auch p-Benzochinon-dioxim oder p-Nitroso-N,N-diethanolanilin eingesetzt werden.

Bei Aufbewahrung im Dunkeln bei Raumtemperatur ist die Verfärbung über mehrere Wochen stabil.

Ersetzt man in obigem Beispiel p-Nitroso-N,N-dimethylanilin durch Peroxidase (100 mg; 200 IU/mg) und Phosphormolybdänsäure durch 3,3',5,5'-Tetramethylbenzidin (300 mg), so erhält man einen sauerstoffabhängigen Glucosetest, wie er prinzipiell aus dem Stand der Technik bekannt ist. Im Gegensatz zu obigem Beispiel muß dann aber die Probelösung nach Aufgabe auf den Teststreifen abgewischt werden, damit Sauerstoff eindiffundieren kann und uberhaupt Färbung eintritt. Zusätzlich zu diesem Nachteil wird die Endfärbung mit höherer Glucosekonzentration immer langsamer erreicht und die erzeugte Farbe ist weniger lagerstabil. Die Vorteile des Ersatzes von Sauerstoff durch erfindungsgemäße Elektronenakzeptoren sind somit:
- kein Abwischen der Probe nach Aufgabe auf den Teststreifen nötig
- schnellere Reaktion
- bei kinetischer Messung von der Analytkonzentration abhängige Reaktionsgeschwindigkeit
- bei Endpunktmessung im Endpunkt von der Analytkonzentration unabhängige Reaktionsgeschwindigkeit
- stabilerer Farbstoff

### Beispiel 6

### Bestimmung von Lactat mit Lactat-Oxidase und Nitrosoverbindungen als Elektronenakzeptor

In einer Küvette wurden 2240 »l Testpuffer (0,2 M Citronensäure/Natriumhydroxid, pH 6,35), 5 »l Elektronenakzeptor (0,1 M N,N-Dimethyl-p-nitroso-anilin in Äthanol) und 250 »l Probe mit bekannten Lactat-Konzentrationen gemischt und auf 25 °C thermostatisiert. Der Test wurde mit 5 »l Enzymlösung (Lactat-Oxidase (pediococcus sp.) 200 U/ml Testpuffer) gestartet und die Extinktionsänderung ΔE/min bei 390 nm registriert. Folgende Ergebnisse wurden erhalten:

| C_{Lactat}:mM | ΔE/min |
|---|---|
| 0 | 0 |
| 0,1 | 0,093 |
| 0,2 | 0,182 |
| 0,3 | 0,250 |
| 0,5 | 0,343 |
| 1,0 | 0,443 |
| 3,0 | 0,508 |

C_{Lactat} ist hierbei die Lactat-Konzentration, die sich bei Durchführung der Messung in der Küvette befindet.

Der Test konnte durch Änderungen von Elektronenakzeptorkonzentration, Enzymkonzentration, Beobachtungswellenlänge und Temperatur beschleunigt oder verlangsamt werden. Als Elektronenakzeptoren konnten auch N,N-Diäthyl-p-nitrosoanilin, N,N-Bis(2-hydroxyäthyl)-p-nitrosoanilin, Benzfuroxan und Resazurin verwendet werden.

## Patentansprüche

1. Verfahren zur Bestimmung eines Analyten mittels enzymatischer Oxidation des Analyten in Gegenwart eines Elektronenakzeptors und Bestimmung des reduzierten Elektronenakzeptors durch Farbbildung als Maß für die Menge des Analyten, dadurch gekennzeichnet, daß der Analyt mit einer flavinabhängigen Oxidase oder einer PQQ-abhängigen Dehydrogenase in Anwesenheit einer aromatischen Nitrosoverbindung oder eines tautomer äquivalenten Oxims als direktem Elektronenakzeptor oxidiert wird.

2. Verfahren gemaß Anspruch 1, dadurch gekennzeichnet, daß als aromatische Nitrosoverbindung eine Verbindung der Formel I 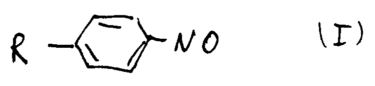 eingesetzt wird, in der R eine Hydroxygruppe oder eine Aminogruppe bedeuten, wobei die Aminogruppe ein- oder zweilach durch Niederalkyl substituiert sein und die Niederalkylgruppe ihrerseits durch eine Hydroxygruppe, eine ein- oder mehrfach durch Niederalkyl substituierte Aminogruppe, PO₃H₂, SO₃H oder CO₂H substituiert sein kann.

3. Verfahren gemaß Anspruch 1 oder 2, dadurch gekennzeichnet, daß die aromatische Nitrosoverbindung p-Hydroxy-Nitrosobenzol, p-Dimethylamino-Nitrosobenzol, p-Diethylamino-Nitrosobenzol oder p-Dihydroryethylamino-Nitrosobenzol darstellt.

4. Verfahren gemäß Anspruch 1 - 3, dadurch gekennzeichnet, daß der reduzierte Elektronenakzeptor durch eine oxidative Kupplungsreaktion durch Farbbildung nachgewiesen wird.

5. Verfahren gemäß Anspruch 1 - 3, dadurch gekennzeichnet, daß der reduzierte Elektronenakzeptor durch Reduktion von Substanzen, die beim Übergang von der oxidierten zur reduzierten Form eine Farbe bilden, nachgewiesen wird.

6. Mittel zur kolorimetrischen Bestimmung eines Analyten durch enzymatische Oxidation des Analyten, enthaltend eine flavinabhängige Oxidase oder eine PQQ-abhängige Dehydrogenase und einen farbbildenden Elektronenakzeptor, dadurch gekennzeichnet, daß der farbbildende Elektronenakzeptor eine direkt mit dem Analyt / Enzymsystem reagierende aromatische Nitrosoverbindung ist.

7. Mittel gemäß Anspruch 6, dadurch gekennzeichnet, daß die aromatische Nitrosoverbindung eine Verbindung der Formel I darstellt, 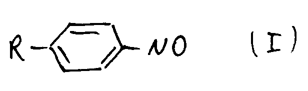 in der
R eine Hydroxygruppe oder eine Aminogruppe bedeuten, wobei die Aminogruppe ein- oder zweifach durch Niederalkyl substituiert sein und die Niederalkylgruppe ihrerseits durch eine Hydrorygruppe, eine ein- oder mehrfach durch Niederalkyl substituierte Aminogruppe, PO₃H₂, SO₃H oder CO₂H substituiert sein kann.

8. Mittel gemäß einem der Ansprüche 6 oder 7, dadurch gekennzeichnet, daß es zusätzlich ein oxidatives Kupplungsreagenz für den reduzierten Elektronenakzeptor enthält.

9. Mittel gemaß einem der Ansprüche 6 oder 7, dadurch gekennzeichnet, daß es zusätzlich eine Substanz enthält, die beim Übergang von der oxidierten zur reduzierten Form eine Farbe bildet.

10. Verwendung einer aromatischen Nitrosoverbindung als direkten farbbildenden Elektronenakzeptor eines Analyt / Enzym-Systems, wobei das Enzym eine flavinabhängige Oxidase oder eine PQQ-abhängige Dehydrogenase darstellt.

## Claims

1. Process for the determination of an analyte by means of enzymatic oxidation of the analyte in the presence of an electron acceptor and determination of the reduced electron acceptor by colour formation as measure for the amount of analyte, characterised in that the analyte is oxidised by a flavine-dependent oxidase or by a PQQ-dependent dehydrogenase in the presence of an aromatic nitroso compound or of a tautomerically equivalent oxide as direct electron acceptor.

2. Process according to claim 1, characterised in that, as aromatic nitroso compound, there is used a compound of the formula I 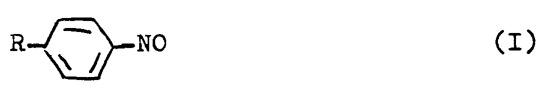 in which R signifies a hydroxyl group or an amino group, whereby the amino group can be substituted once or twice by lower alkyl and the lower alkyl group can in turn be substituted by a hydroxyl group, an amino group substituted one or more times by lower alkyl, PO₃H₂, SO₃H or CO₂H.

3. Process according to claim 1 or 2, characterised in that the aromatic nitroso compound represents p-hydroxynitrosobenzene, p-dimethylaminonitrosobenzene, p-diethylaminonitrosobenzene or p-dihydroxyethylaminonitrosobenzene.

4. Process according to claim 1 - 3, characterised in that the reduced electron acceptor is detected by an oxidative coupling reaction by colour formation.

5. Process according to claim 1 - 3, characterised in that the reduced electron acceptor is detected by reduction of substances which form a colour in the case of changing from the oxidised to the reduced form.

6. Agent for the colorimetric determination of an analyte by enzymatic oxidation of the analyte, containing a flavine-dependent oxidase or a PQQ-dependent dehydrogenase and a colour-forming electron acceptor, characterised in that the colour-forming electron acceptor is an aromatic nitroso compound reacting directly with the analyte/enzyme system.

7. Agent according to claim 6, characterised in that the aromatic nitroso compound represents a compound of the formula I 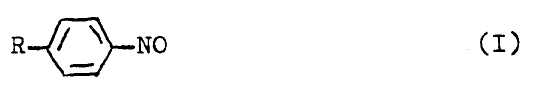 in which R signifies a hydroxyl group or an amino group, whereby the amino group can be substituted once or twice by lower alkyl and the lower alkyl group can in turn be substituted by a hydroxyl group, an amino group substituted one or more times by lower alkyl, PO₃H₂, SO₃H or CO₂H.

8. Agent according to one of claims 6 or 7, characterised in that it additionally contains an oxidative coupling reagent for the reduced electron acceptor.

9. Agent according to one of claims 6 or 7, characterised in that it additionally contains a substance which forms a colour in the case of changing from the oxidised to the reduced form.

10. Use of an aromatic nitroso compound as direct colour-forming electron acceptor of an analyte/enzyme system, whereby the enzyme represents a flavine-dependent oxidase or a PQQ-dependent dehydrogenase.

## Revendications

1. Procédé de détermination d'un analyte par oxydation enzymatique de l'analyte en présence d'un accepteur d'électrons et détermination de l'accepteur d'électrons réduit par formation d'une couleur comme mesure de la quantité de l'analyte, caractérisé en ce que l'analyte est oxydé au moyen d'une oxydase flavine-dépendante ou d'une déshydrogénase PQQ-dépendante, en présence d'un composé nitroso aromatique ou d'un oxime équivalent tautomère en tant qu'accepteur d'électrons direct.

2. Procédé selon la revendication 1, caractérisé en ce que, comme composé nitroso aromatique, on utilise un composé de formule I 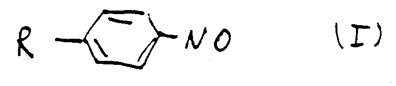 dans laquelle R signifie un groupe hydroxy ou un groupe amino, le groupe amino pouvant être substitué une ou deux fois par un groupe alkyle inférieur, et le groupe alkyle inférieur pouvant être substitué à son tour par un groupe hydroxy, un groupe amino substitué une ou plusieurs fois par un groupe alkyle inférieur, un groupe PO₃H₂, SO₃H ou CO₂H.

3. Procédé selon la revendication 1 ou 2, caractérisé en ce que le composé nitroso aromatique représente le p-hydroxy-nitrosobenzène, le p-diméthylamino-nitrosobenzène, le p-diéthylamino-nitrosobenzène ou le p-dihydroxyéthylamino-nitrosobenzène.

4. Procédé selon les revendications 1-3, caractérisé en ce que l'accepteur d'électrons réduit est détecté par une réaction de couplage oxydante chromogène.

5. Procédé selon les revendications 1-3, caractérisé en ce que l'accepteur d'électrons réduit est détecté par réduction de substances dont le passage de la forme oxydée à la forme réduite est chromogène.

6. Moyen pour la détermination colorimétrique d'un analyte par oxydation enzymatique de l'analyte, contenant une oxydase flavine-dépendante ou une déshydrogénase PQQ-dépendante, et un accepteur d'électrons chromogène, caractérisé en ce que l'accepteur d'électrons chromogène est un composé nitroso aromatique réagissant directement avec le système analyte/enzyme.

7. Moyen selon la revendication 6, caractérisé en ce que le composé nitroso aromatique représente un composé de formule I 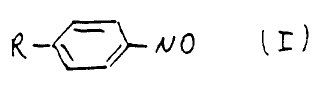 dans laquelle R signifie un groupe hydroxy ou un groupe amino, le groupe amino pouvant être substitué une ou deux fois par un groupe alkyle inférieur, et le groupe alkyle inférieur pouvant être substitué à son tour par un groupe hydroxy, un groupe amino substitué une ou plusieurs fois par un groupe alkyle inférieur, un groupe PO₃H₂, SO₃H ou CO₂H.

8. Moyen selon l'une des revendications 6 ou 7, caractérisé en ce qu'il contient en outre un réactif de couplage oxydant pour l'accepteur l'électrons réduit.

9. Moyen selon l'une des revendications 6 ou 7, caractérisé en ce qu'il contient en outre une substance dont le passage de la forme oxydée à la forme réduite est chromogène.

10. Utilisation d'un composé nitroso aromatique comme accepteur d'électrons chromogène direct d'un système analyte/enzyme, l'enzyme représentant une oxydase flavine-dépendante ou une déshydrogénase PQQ-dépendante.
